**(19)** Europäisches Patentamt

European Patent Office

Office européen des brevets

**(11)** **EP 1 138 761 A1**

**(12)** **EUROPEAN PATENT APPLICATION**
published in accordance with Art. 158(3) EPC

**(43)** Date of publication:
**04.10.2001 Bulletin 2001/40**

**(51)** Int Cl.⁷: **C12N 15/00**, C12M 1/00,
C12Q 1/68, G01N 33/53,
G01N 33/50

**(21)** Application number: **99959690.1**

**(22)** Date of filing: **08.12.1999**

**(86)** International application number:
**PCT/JP99/06865**

**(87)** International publication number:
**WO 00/34456 (15.06.2000 Gazette 2000/24)**

**(84)** Designated Contracting States:
**AT BE CH CY DE DK ES FI FR GB GR IE IT LI LU
MC NL PT SE**
Designated Extension States:
**AL LT LV MK RO SI**

**(30)** Priority: **10.12.1998 JP 35127698**

**(71)** Applicant: **Takara Shuzo Co, Ltd.
Kyoto-shi, Kyoto 612-8061 (JP)**

**(72)** Inventors:
 • **TAKAYAMA, Masanori
  Kyotanabe-shi, Kyoto 610-0311 (JP)**
 • **ROKUSHIMA, Masatomo
  Otsu-shi, Shiga 520-2133 (JP)**
 • **UEDA, Minoru 412, Hamoparesu-Kusatsu
  Kusatsu-shi, Shiga 525-0025 (JP)**

 • **OKAMOTO, Sachiko
  Joyo-shi, Kyoto 610-0121 (JP)**
 • **OZAKI, Aya
  Otsu-shi, Shiga 520-0037 (JP)**
 • **MINENO, Junichi
  Uji-shi, Kyoto 611-0002 (JP)**
 • **KIMIZUKA, Fusao
  Omihachiman-shi, Shiga 523-0056 (JP)**
 • **ASADA, Kiyozo
  Koka-gun, Shiga 520-3333 (JP)**
 • **KATO, Ikunoshin
  Uji-shi, Kyoto 611-0028 (JP)**

**(74)** Representative: **Grund, Martin, Dr. et al
Dr. Volker Vossius,
Patentanwaltskanzlei,
Holbeinstrasse 5
81679 München (DE)**

**(54) METHOD FOR IMMOBILIZING DNA ON A CARRIER**

**(57)** A method for immobilizing DNA on a carrier characterized by having the step of bringing the DNA into contact with the carrier in a buffer containing one or more members selected from the group consisting of morpholine, morpholine derivatives, salts thereof and carbonates thereof.

EP 1 138 761 A1

**Description**

Technical Field

The present invention relates to a method for immobilizing a DNA onto a solid support, which is useful for preparation of a DNA chip and the like. The present invention also relates to a material onto which a DNA is immobilized according to said method and a method for detecting a target nucleic acid using said material.

Background Art

With the progress of projects for analyzing genomes of organisms including human, the structures of genomes of various organisms have gradually become elucidated. In parallel, techniques for analyzing the functions of the genomes have been developed in order to utilize the vast information on the genomes for the development of pharmaceuticals or the maintenance or improvement of health. DNA chip technology is one of the principal techniques noticeable for this purpose. A DNA chip is a microarray (DNA array) in which a number of various genes or DNA fragments are arrayed and immobilized onto a solid support such as a slide glass. The DNA chip is very useful as means to greatly accelerate the analysis of expression, mutation or polymorphism of a gene.

It is necessary to immobilize a DNA onto a support in order to prepare a DNA chip. Known methods for immobilizing a DNA is generally classified into two groups.

One is a group of methods in which a DNA is chemically synthesized on a tip of a linker covalently bonded to a support, for example, as described in Science, 251:767-773 (1991) and Nucleic Acids Research, 20:1679-1684 (1992). Such a method can be used to immobilize a DNA (an oligonucleotide) which can be synthesized and of which the sequence is known. It is said that the yield in each step of this method is lower than that of conventional solid phase synthesis. Furthermore, the method requires a micro apparatus for completely sealing reaction sites.

The other is a group of methods in which a DNA which has been synthesized beforehand or a DNA which has been prepared by polymerase chain reaction (PCR) (a PCR amplification product) is covalently or non-covalently (or electrostatically) bonded to a support as described, for example, in Science, 270:467-470 (1995) and Nucleic Acids Research, 22:5456-5465 (1994). It is supposed that one can immobilize any DNAs according to such a method. However, for example, in case of non-covalent bonding, it is difficult to immobilize a short chain DNA (an oligonucleotide) such as a synthetic DNA. If a long chain DNA such as a PCR product is to be immobilized onto a support via a non-covalent bond, the DNA is generally immobilized by denaturing the DNA dissolved in a salt solution such as sodium chloride/sodium citrate (SSC), tris-hydrochloride/EDTA (TE) or phosphate buffered saline (PBS), and then spotting it onto a slide glass (a support) coated with a polycation such as polylysine or polyethylenimine. In this case, it is known that a considerable amount of the DNA is detached from the support during the subsequent step of washing or hybridization with a target nucleic acid. This phenomenon is a factor that reduces the sensitivity of detecting a target nucleic acid.

It is necessary to heat- or alkali-denature a DNA beforehand in order to increase the immobilization efficiency to a support. It is known that the immobilization efficiency is remarkably reduced in the absence of denaturing. In addition, if a number of DNA solutions are to be immobilized onto a slide glass using an instrument for preparing DNA chips (an arrayer), the denaturing step make the entire steps complicated, making it difficult to automate the procedure.

The absolute quantity of an immobilized DNA is increased by increasing the concentration of the DNA in a solution to be spotted onto a support. However, as a result, the immobilization rate to the support is decreased and the loss of the DNA used is increased.

When an instrument for preparing DNA chips (an arrayer) is used, the volume of a DNA solution to be spotted for the preparation of a DNA array may be less than 1 nanoliter. Thus, a certain DNA solution may be dried before it diffuses on the surface of the support, resulting in formation of heterogeneous dots.

Additionally, in case of covalent bonding, it is necessary to introduce an appropriate functional group into a DNA beforehand. The introduction procedure is complicated.

If a target nucleic acid is detected using a DNA chip or the like, the detection sensitivity depends on the amount of immobilized DNA in a unit area (or in a dot) . Thus, the way of increasing the amount of immobilized DNA in a dot, in other words, the way of increasing the density of an immobilized DNA, has become a very important problem. Furthermore, the way of spotting the DNA solution while allowing it to diffuse homogeneously has also become very important.

Objects of Invention

The main object of the present invention is to provide (1) an effective method for immobilizing a DNA onto a solid support, (2) a material onto which a DNA is immobilized prepared according to said method, and (3) a method for detecting a target nucleic acid using said material.

Summary of Invention

The present inventors have studied intensively and found that the amount of an immobilized DNA in a dot area or a unit area is greatly increased, which DNA is dissolved in a buffer containing at least one substance selected from the group consisting of morpholine, a morpholine derivative, a salt thereof and a carbonate and then spotted onto a support. The present inventors have also found that a DNA solution can be spotted onto a support while allowing it to diffuse homogeneously on the surface of the support by adding a surfactant in the buffer. The present inventors have further found that sensitivity of detecting a target nucleic acid by hybridization under stringent conditions is increased as a result of increase in a DNA immobilization rate and increase in DNA immobilization density in a unit area by the use of the immobilization techniques. The present inventors have constructed an excellent method for detecting a target nucleic acid by combining these techniques. Thus, the present invention has been completed.

Accordingly, the present invention provides the following:

(1) a method for immobilizing a DNA onto a support, the method comprising contacting a DNA with a support in a buffer containing at least one substance selected from the group consisting of morpholine, a morpholine derivative, a salt thereof and a carbonate;

(2) the method according to (1) above, wherein the morpholine derivative is an N-alkylmorpholine substituted with a C1-3 alkyl group;

(3) the method according to (1) above, wherein the salt of morpholine or the morpholine derivative is a salt selected from the group consisting of a salt with a mineral acid, a salt with an organic acid and a salt with a fatty acid;

(4) the method according to (1) above, wherein the carbonate is a salt selected from the group consisting of a sodium salt, a potassium salt, a magnesium salt, an ammonium salt and a triethylamine salt;

(5) the method according to (1) above, wherein the buffer contains at least one substance selected from the group consisting of morpholine, a morpholine derivative and a salt thereof in combination with at least one carbonate;

(6) the method according to any one of (1) to (5) above, wherein the pH of the buffer is 7 to 11;

(7) the method according to any one of (1) to (6) above, wherein the concentration of at least one substance selected from the group consisting of morpholine, a morpholine derivative, a salt thereof and a carbonate in the buffer is 10 to 500 mM;

(8) the method according to any one of (1) to (7) above, wherein the DNA is an oligonucleotide, a polynucleotide or a derivative thereof;

(9) the method according to any one of (1) to (8) above, wherein the concentration of the DNA in the buffer is 0.1 to 2.0 mg/ml;

(10) the method according to any one of (1) to (9) above, wherein the buffer further contains at least one salt;

(11) the method according to any one of (1) to (10) above, wherein the buffer further contains at least one surfactant;

(12) the method according to any one of (1) to (11) above, wherein the support is made from glass or quartz, or is a material prepared by treating the surface of glass or quartz;

(13) the method according to (12) above, wherein the surface is treated with a silane coupling agent or a polycation;

(14) the method according to any one of (1) to (13) above, wherein the DNA is immobilized without denaturation;

(15) the method according to (14) above, wherein the DNA is a double-stranded DNA;

(16) a method for immobilizing a DNA onto a support, the method comprising contacting a DNA with a support in a buffer containing at least one surfactant;

(17) the method according to (16) above, wherein the surfactant is selected from the group consisting of a non-ionic surfactant, an anionic surfactant and an amphoteric surfactant;

(18) the method according to (17) above, wherein the surfactant is selected from the group consisting of sucrose monocaprate, sucrose monolaurate and digitonin;

(19) the method according to any one of (16) to (18) above, wherein the buffer contains at least one substance selected from the group consisting of morpholine, a morpholine derivative, a salt thereof and a carbonate;

(20) the method according to (19) above, wherein the pH of the buffer is 7 to 11;

(21) the method according to (19) or (20) above, wherein the concentration of at least one substance selected from the group consisting of morpholine, a morpholine derivative, a salt thereof and a carbonate in the buffer is 10 to 500 mM;

(22) the method according to any one of (16) to (21) above, wherein the DNA is an oligonucleotide, a polynucleotide or a derivative thereof;

(23) the method according any one of (16) to (22) above, wherein the concentration of the DNA in the buffer is 0.1 to 2.0 mg/ml;

(24) the method according to any one of (16) to (23) above, wherein the buffer further contains at least one salt;

(25) the method according to any one of (16) to (24) above, wherein the support is made from glass or quartz, or is a material prepared by treating the surface of glass or quartz;

(26) the method according to (25) above, wherein the surface is treated with a silane coupling agent or a polycation;

(27) the method according to any one of (16) to (26) above, wherein the DNA is immobilized without denaturation;

(28) the method according to (27) above, wherein the DNA is a double-stranded DNA;

(29) a material onto which a DNA is immobilized, which is prepared according to the method defined by any one of (1) to (28) above;

(30) the material according to (29) above, wherein the DNA is a double-stranded DNA;

(31) a method for detecting a target nucleic acid, the method comprising detecting a target nucleic acid by using the material onto which a DNA is immobilized defined by (29) or (30) above;

(32) the method according to (31) above, comprising hybridizing the material onto which a DNA is immobilized with the target nucleic acid under stringent conditions;

(33) the method according to (32) above, wherein the DNA immobilized on the material is hybridized with the target nucleic acid under stringent conditions without denaturation of the immobilized DNA;

(34) a material in which a double-stranded DNA is immobilized onto a support, which can be used for hybridization with a target nucleic acid under stringent conditions without denaturing the immobilized DNA;

(35) a buffer for immobilizing a DNA, which contains at least one substance selected from the group consisting of morpholine, a morpholine derivative, a salt thereof and a carbonate;

(36) a buffer for immobilizing a DNA, which contains at least one surfactant; and

(37) a buffer for immobilizing a DNA, which contains at least one substance selected from the group consisting of morpholine, a morpholine derivative, a salt thereof and a carbonate as well as at least one surfactant.

Brief Description of Drawings

Figure 1: A figure which illustrates the effect of the addition of a surfactant on spotting.
Figure 2: A figure which illustrates the effect of the addition of a surfactant on spotting.

Detailed Description of the Invention

A buffer containing at least one substance selected from the group consisting of morpholine, a morpholine derivative, a salt thereof and a carbonate in the present invention (hereinafter simply referred to as an essential buffering component) may be, for example, a buffer commonly used for biochemical studies which contains such an essential buffering component.

Examples of morpholine, morpholine derivatives and salts thereof as essential buffering components include, but are not limited to, morpholine, an N-alkylmorpholine substituted with a C1-3 alkyl group (e.g., N-methylmorpholine, N-ethylmorpholine or N-propylmorpholine), as well as a salt thereof with a mineral acid (e.g., hydrochloric acid or carbonic acid), an organic acid (e.g., acetic acid, lactic acid or citric acid) or a fatty acid (e.g., lauric acid or stearic acid).

Carbonates as essential buffering components include, but are not limited to, a salt of carbonic acid with an alkaline metal, an alkaline earth metal, ammonium or an organic amine such as sodium carbonate, sodium hydrogencarbonate, potassium carbonate, potassium hydrogencarbonate, magnesium carbonate, ammonium carbonate or triethylamine carbonate.

Such an essential buffering component can be used alone, or two or more components can be used in combination. In one embodiment, any one of morpholine, morpholine derivatives and salts thereof and any one of carbonates are used in combination.

The pH of the buffer used in the present invention is adjusted to preferably 7 to 11, more preferably 8 to 10.

The salt concentration of the buffer is preferably 10 to 500 mM, more preferably 50 to 200 mM.

In the present invention, the buffer may further contain other salts such as sodium chloride in addition to the above-mentioned essential buffering component. The buffer of the present invention may contain one or more surfactant. The surfactant to be used is not limited to specific one. Any surfactants can be preferably used as long as the homogeneous diffusion of the DNA solution on the surface of the support is regulated by the addition thereof.

In another embodiments, the present invention provides a method for immobilizing a DNA onto a support, the method comprising contacting a DNA with a support in a buffer containing at least one surfactant.

The surfactant to be used in this method is not limited to specific one. Any surfactants can be preferably used as long as the homogeneous diffusion of the DNA solution on the surface of the support is regulated by the addition thereof upon immobilization.

Surfactants are classified into non-ionic surfactants, cationic surfactants, anionic surfactants, amphoteric surfactants and the like based on their ingredients. Either of them can be used in the present invention as long as they have the above-mentioned activities. For example, the following surfactants can be used in the present invention. Non-ionic: Nonidet P-40, Triton X-100, Tween-20, n-octyl-β-D-glucoside, n-heptyl-(β-D-thioglucoside, n-octyl-β-D-thioglucoside, n-dodecyl-β-D-maltoside, sucrose monocaprate, sucrose monolaurate, digitonin, heptanoyl-N-methylglucamide (MEGA-7), octanoyl-N-methylgucamide (MEGA-8), nonanoyl-N-methylglucamide (MEGA-9), decanoyl-N-methylglucamide (MEGA-10), N,N-bis[3-D-gluconamidopropyl]cholamide (BIGCHAP) and N,N-bis[3-D-gluconamidopropyl] deoxycholamide (deoxy-BIGCHAP). Anonic: deoxycholic acid, sodium cholate, Sarkosyl and sodium dodecyl sulfate (SDS). Amphoteric: 3-[3-(cholamidopropyl)dimethylammonio]-1-propanesulfonate (CHAPS) and 3-[3-(cholamidopropyl)dimethylammonio]-2-hydroxy-1-propanesulfonate (CHAPSO). In particular, the non-ionic surfactants are preferably used in the present invention.

The surfactant may be used at a concentration within a range that results in homogeneous diffusion of a DNA solution on a surface of a support. For example, the surfactant can be used at a final concentration of 0.001 to 0.1%, although it is not intended to limit the present invention.

The buffer used for the immobilization of a DNA onto a support in the presence of a surfactant is not limited to specific one as long as it is suitable for immobilization. For example, a buffer containing at least one substance selected from the group consisting of morpholine, a morpholine derivative, a salt thereof and a carbonate is preferably used. The type, concentration, pH and the like of the buffering component in the buffer which can be used are as described above. They may be appropriately selected depending on the DNA to be immobilized and the support to be used.

A DNA solution is homogeneously diffused on a surface of a support upon immobilization by using a DNA dissolved in a buffer containing the above-mentioned surfactant for immobilization onto a support, resulting in reduced dispersion of formed dots. When a material onto which a DNA is immobilized prepared as described above is used for detecting a target nucleic acid, more reliable and quantitative data than conventional one can be obtained. The amount of a DNA immobilized on a support is increased by the addition of a surfactant, making it possible to detect a target nucleic acid with higher sensitivity.

A buffer for immobilizing a DNA containing a component as described above is encompassed by the present invention.

As used herein, DNAs include, but are not limited to, a polynucleotide extracted from a cell or a tissue, a DNA enzymatically synthesized in vitro such as a PCR amplification product, a chemically synthesized oligonucleotide and a derivative thereof. The DNA may be single-stranded or double-stranded. As used herein, an oligonucleotide refers to a relatively short DNA. Although it is not intended to limit the present invention, an oligonucleotide means a DNA of 6 to 100 bases in length. Preferably, a DNA of 8 to 50 bases in length is used as an oligonucleotide in the present invention. A polynucleotide refers to a DNA longer than an oligonucleotide herein.

The derivative is not limited to specific one as long as it has modification which enables immobilization onto a surface of a support. A DNA having a functional group such as an amino group or a thiol group being introduced at the 5'-terminus is exemplified.

The DNA to be immobilized onto a support in the present invention is not limited to a specific one as long as it can be hybridized with a target nucleic acid. Both of a single-stranded DNA and a double-stranded DNA can be preferably utilized. When a double-stranded DNA is used, it may be used for hybridization with a target nucleic acid after denaturing into single-stranded DNAs by appropriate means.

The concentration of the DNA to be immobilized is preferably 0.05 to 5.0 mg/ml, more preferably 0.1 to 2.0 mg/ml.

A support used for immobilizing a DNA in the present invention is not limited to specific one as long as it is made from a substantially insoluble material. Examples of the materials include paper, nylon, cellulose, cellulose derivatives such as nitrocellulose, plastics and plastic derivatives such as polycarbonate, polystyrene and polypropylene, magnetic or non-magnetic metals, quartz and glass. The materials may be porous, smooth-surfaced, polymeric or non-polymeric.

The supports to be used in the present invention are not limited to those made from such materials. Any supports which can be used as carries for DNA chips or biosensors can be used as long as they can retain DNAs to be immobilized on their surfaces. For example, a support having a hydrophilic or hydrophobic functional group such as a hydroxyl group, an amine group, a thiol group, an aldehyde group, an epoxy group, a carboxyl group or an acyl group on its surface can be preferably utilized. Such a functional group can be utilized as it is or as an appropriate derivative. For example, in case of a carboxyl group, it can be used as an activated ester derivative such as an N-hydroxysuccinimide ester.

The supports include those having the functional groups on their surfaces as the properties of the materials for the supports.

A support that does not have a hydrophilic or hydrophobic functional group on the surface of the support can be used without limitation if it can be made to have a hydrophilic or hydrophobic functional group on its surface by treating the surface.

Examples of such supports prepared by surface treatment include those prepared by treating glass or quartz with a commercially available silane coupling agent such as aminoalkylsilane, with a polycation such as polylysine or polyethylenimine, or with aminoalkylsilane and glutaraldehyde. A slide glass having a functional group as described above being introduced is commercially available from Sigma.

The method for immobilizing a DNA onto a support of the present invention is described in detail below.

A DNA to be immobilized is dissolved, as it is or after alkali or heat denaturation, in a buffer containing the above-mentioned essential buffering component. The buffer may optionally contain a neutral salt and/or a surfactant.

A constant volume of the resulting buffer containing the DNA is spotted onto a support (e.g., a slide glass) using a micropipette, a microdispenser or an instrument for preparing DNA chips. The support onto which the DNA has been spotted is incubated, for example, for 1 hour in a humidified incubator. The DNA is then crosslinked by ultraviolet irradiation. The support is washed in a 0.2% sodium dodecyl sulfate (SDS) aqueous solution followed by distilled water and dried.

Detachment of a DNA immobilized according to the above-mentioned immobilization method during a step of washing or hybridization can be made less than that for a conventional method. Furthermore, a DNA immobilization rate in a dot area or a unit area can be increased by using the immobilization method. In other words, the density of a DNA immobilized in a unit area can be increased. Thus, the sensitivity of detecting a target nucleic acid can be increased as compared with conventional methods by using the support having DNAs immobilized according to the above-mentioned method.

Furthermore, immobilization can be conducted without denaturation in the method of the present invention with an efficiency equivalent to that for immobilization with denaturation, which denaturation prior to spotting is indispensable for conventional methods.

An immobilization rate of a DNA to a support can be evaluated by immobilizing a DNA labeled with a fluorescent substance (e.g., Rhodamine) onto a support, and measuring the intensity of fluorescence emitted from a dot on the support using a fluorescence image analyzer such as FMBIO II Multi-View (Takara Shuzo). The immobilization rate can be calculated according to equation 1 below.

Equation 1:

Immobilization rate (%) = 100 x (fluorescence intensity for a dot on a support after immobilization and washing) / (fluorescence intensity for a dot immediately after immobilization)

The density of an immobilized DNA can be determined, for example, as follows. A DNA labeled with a fluorescent substance (e.g., Rhodamine) is immobilized. The intensity of fluorescence emitted from a dot in which the DNA is immobilized can be measured using a fluorescence image analyzer such as FMBIO II Multi-View. The amount of the DNA immobilized in the dot can be calculated on the basis of a calibration curve prepared for the amount of fluorescence-labeled DNA used and the fluorescence intensity.

The amount of a DNA immobilized in a unit area, that is, the density of an immobilized DNA, can be determined by measuring the area of the dot, for example, using the fluorescence image analyzer.

Alternatively, a predetermined amount of a DNA solution is spotted onto a support according to the DNA immobilization method of the present invention, and the immobilization rate is determined. The amount of an immobilized DNA can be calculated based on the determined immobilization rate and the amount of the spotted DNA. The area of a dot can be determined, for example, by using a microscope. The density of the immobilized DNA can also be determined based on the amount of the immobilized DNA and the area determined as described above.

A material onto which a DNA is immobilized obtained as described above is also encompassed by the present invention.

A method of detecting a target nucleic acid using the material onto which a DNA is immobilized is also encompassed by the present invention. In this method, the material onto which a DNA is immobilized is hybridized with a target nucleic acid under stringent conditions.

As used herein, stringent conditions refer to those as described in Molecular cloning, A laboratory manual, 2nd ed., pp. 9.47-9.51 (1989, Cold Spring Harbor Laboratory). For example, a material onto which an oligonucleotide as a DNA is immobilized is hybridized with a target nucleic acid under stringent conditions as follows, although the conditions may vary depending on, among others, the length of the base sequence in a portion of the target nucleic acid that hybridizes with the immobilized DNA. A Tm value is calculated based on the length of the portion to be hybridized. Hybridization is carried out at a temperature lower than the calculated Tm value by 20 to 25°C in a solution having a high ionic strength (e.g., 6 x SSC or 6 x SSPE). Usually, it is preferable to conduct washing at a temperature lower than the Tm value by 12 to 20°C while changing the salt concentrations of washing buffers in a subsequent washing step.

For a material onto which a DNA other than an oligonucleotide, i.e., a polynucleotide, is immobilized, stringent conditions refer to those as described in Molecular cloning, A laboratory manual, 2nd ed., pp. 9.52-9.55 (1989) and are exemplified by hybridization of a polynucleotide with a target nucleic acid and washing at 68°C, without limitation.

The target nucleic acid is not limited to specific one. For example, any nucleic acids can be used as a target to screen a DNA that hybridizes with the target nucleic acid among DNAs immobilized onto a support.

According to the present invention, a support onto which a DNA has been immobilized without denaturing the DNA can be used for hybridization without denaturation.

Furthermore, the present invention provides a material in which a double-stranded DNA is immobilized onto a support and which can be used for hybridization with a target nucleic acid under stringent conditions without alkali- or heat-denaturing the immobilized DNA.

Such a material can be prepared according to a method in which a double-stranded DNA dissolved, for example, in morpholine buffer or carbonate buffer is spotted or the like, although it is not intended to limit the present invention. All materials in which double-stranded DNAs are immobilized onto supports and which can be used for hybridization with a target nucleic acid under stringent conditions without denaturation are encompassed by the present invention.

Thus, a series of steps from the immobilization of a DNA into a support to the detection of a target nucleic acid using the material can be simplified by using the method for immobilizing a DNA onto a support of the present invention or the material onto which a DNA is immobilized of the present invention.

Such simplification of steps results in not only increase in operational efficiency in view of time or reagents used but also decrease in detachment of a DNA from a support due to denaturation as well as increase in sensitivity of detecting a target nucleic acid.

As described above, by using the method for immobilizing a DNA onto a support of the present invention, the amount of a DNA immobilized in a unit area on a support can be increased, the immobilization rate or the density of an immobilized DNA can be increased. A material onto which a DNA is immobilized, in which the amount of a DNA immobilized in a unit area on a support is increased and the immobilization rate or the density of an immobilized DNA is increased, can be provided according to said method. Furthermore, sensitivity of detecting a target nucleic acid can be increased by using said material.

Examples

The following examples further illustrate the present invention in detail but are not to be construed to limit the scope thereof.

Example 1

Effect of solvent for DNA on immobilization of PCR amplification product

(1) Preparation of fluorescence-labeled PCR amplification product

A plasmid DNA containing human transferrin receptor gene (GenBank accession number X01060, 4738 bp) was used as a template to amplify a fragment of about 1 kb for the gene by a PCR. The base sequences of two primers used for the PCR are shown in SEQ ID NOS:1 and 2. The primer of SEQ ID NO:1 was used after fluorescence-labeling with Rhodamine X (PE Biosystems, hereinafter referred to as ROX) at the 5'-terminus.

(2) Preparation of buffer for test and method for immobilizing DNA

Solutions prepared so as to have the following compositions were diluted to given concentrations and used as various buffers for tests as shown in Table 1. 20 x SSC: 0.3 M sodium citrate containing 3M sodium chloride. TE: 10 mM tris-hydrochloride (pH 8.0) containing 1 mM EDTA. PBS: prepared using a PBS tablet (Takara Shuzo). 100 mM citrate buffer: prepared by mixing a 100 mM citric acid solution with a 100 mM sodium citrate solution to adjust the pH to a given value. 500 mM carbonate buffer: prepared by mixing a 500 mM sodium carbonate solution and a 500 mM sodium hydrogencarbonate solution to adjust the pH to a given value. 500 mM morpholine buffer: prepared by adjusting the pH of 500 mM N-methylmorpholine to a given value with diluted hydrochloric acid. 50 mM borate buffer: prepared by adjusting the pH of a 50 mM boric acid solution containing 50 mM potassium chloride to a given value with 2 N sodium hydroxide.

The fluorescence-labeled PCR amplification product was dissolved in each of the solutions at a concentration of 0.7 mg/ml (1 μM). The resulting solutions were used as DNA solutions. 0.2 μl each of the DNA solutions was spotted onto a slide glass treated with aminoalkylsilane (Sigma) with or without denaturation. The slide glass was air-dried and then incubated at 37°C for 1 hour in a humidified incubator.

The slide glass was irradiated with ultraviolet at 60 mJ, and then soaked in a solution for 15 minutes to block free amino groups. The solution was prepared by dissolving 5 g of succinic anhydride (Nacalai Tesque) in 315 ml of N-methyl-2-pyridone (Nacalai Tesque) and further adding 35 ml of 0.2 M borate buffer (pH 8.0) thereto. Finally, the slide glass was washed in a 0.2% SDS solution for 2 minutes followed by distilled water and dried at room temperature. The slide glass was subjected to stringent hybridization conditions as follows. 4 x SSC containing 0.2% SDS was spotted onto the region of the spotted DNAs on the slide glass. The spot was covered with a cover glass such that no air bubble was included. The sides were shut tightly with a seal and the slide glass was incubated at 37 to 40°C for 2 to 6 hours. After removing the seal, the slide glass was washed in 0.2 x SSC containing 0.1% SDS for 30 minutes followed by 0.2 x SSC for 30 minutes and dried.

(3) Determination of immobilization rate

Fluorescence intensities were measured immediately after immobilization and after treatment under hybridization conditions using a fluorescence image analyzer FMBIO II Multi-View. Immobilization rates were calculated based on the values of the respective fluorescence intensities. The results are shown in Table 1.

Table 1

| Solvent for DNA | Immobilization rate (%) |
|---|---|
| 3 x SSC | 2.3 |
| 1 x SSC | 1.7 |
| TE (pH8.0) | 5.5 |
| PBS (pH7.6) | 4.7 |
| 100mM Citrate buffer (pH4.0) | 8.5 |
| 100mM Citrate buffer (pH6.0) | 3.9 |
| 100mM Glycine buffer (pH9.5) | 1.8 |
| 50mM Carbonate buffer (pH9.5) | 30.2 |
| 50mM Morpholine buffer (pH9.0) | 58.6 |
| 50mM Borate buffer (pH9.0) | 2.6 |

As shown in Table 1, it was confirmed that the immobilization rate observed when a DNA was spotted in morpholine buffer or carbonate buffer was remarkably increased as compared with that observed using a conventional solvent SSC, PBS or TE.

Example 2

Effect of pH and buffer salt concentration of morpholine buffer and carbonate buffer on immobilization of PCR amplification product

(1) Effect of pH of morpholine buffer and carbonate buffer on immobilization of PCR amplification product

N-methylmorpholine hydrochloride (NMM in Table 2) buffer as morpholine buffer and carbonate buffer were used.

The PCR amplification product was dissolved in each of NMM buffers (pH 7-10) and carbonate buffers (pH 9.5-10.5). Immobilization was carried out as described in Example 1. The immobilization rates were determined based on the fluorescence intensities. The results are shown in Table 2.

Table 2

| Solvent for DNA | | Immobilization rate (%) |
|---|---|---|
| Buffer | pH | |
| 50mM NMM buffer | 7.0 | 41.2 |
| | 7.5 | 46.5 |
| | 8.0 | 41.0 |
| | 8.5 | 43.2 |
| | 9.0 | 58.6 |
| | 9.5 | 47.0 |
| | 9.9 | 27.9 |
| 50mM Carbonate buffer | 9.5 | 30.2 |
| | 10.0 | 25.8 |
| | 10.5 | 20.8 |

Table 2

| Solvent for DNA | | Immobilization rate (%) |
|---|---|---|
| Buffer | pH | |
| 50mM NMM buffer | 7.0 | 41.2 |
| | 7.5 | 46.5 |
| | 8.0 | 41.0 |
| | 8.5 | 43.2 |
| | 9.0 | 58.6 |
| | 9.5 | 47.0 |
| | 9.9 | 27.9 |
| 50mM Carbonate buffer | 9.5 | 30.2 |
| | 10.0 | 25.8 |
| | 10.5 | 20.8 |

As shown in Table 2, a high immobilization rate was observed for NMM buffer having a wide range of pH (7.0 to 9.9). For carbonate buffer, the immobilization rate was confirmed to be stable at a wide range of pH (9.5 to 10.5).

(2) Effect of salt concentration of morpholine buffer and carbonate buffer on immobilization of PCR amplification product

NMM buffer (pH 9.5) and carbonate buffer (pH 9.5) as described in (1) above were used for examination. The salt concentrations of the respective buffers examined were as shown in Table 3. Immobilization and determination of immobilization rates were as described in (1) above. The results are shown in Table 3.

Table 3

| Solvent for DNA | | Immobilization rate (%) |
|---|---|---|
| Buffer | Concentration (mM) | |
| NMM buffer (pH9.5) | 10 | 73.9 |
| | 50 | 64.8 |
| | 100 | 67.7 |
| | 200 | 58.9 |
| | 500 | 49.8 |
| Carbonate buffer (pH9.5) | 10 | 41.6 |
| | 50 | 37.8 |
| | 100 | 28.4 |

Table 3   (continued)

| Solvent for DNA | | Immobilization rate (%) |
|---|---|---|
| Buffer | Concentration (mM) | |
| | 200 | 44.7 |

As shown in Table 3, a high immobilization rate was observed for NMM buffer having a wide range of salt concentration (10 mM to 500 mM). Also, a high immobilization rate was observed for carbonate buffer having a wide range of salt concentration (10 mM to 200 mM).

Example 3

Effect of surface treatment of support on immobilization of PCR amplification product

The PCR amplification product was dissolved in one of 50 mM N-methylmorpholine hydrichloride (NMM) buffers (pH 8.0 or 9.0) or 50 mM carbonate buffers (pH 9.5 or 10.5) at a concentration of 1 μM. 0.2 μl each of the resulting solutions was spotted onto slide glasses which had been subjected to various surface treatment as shown in Table 4, i.e., a slide glass treated with aminoalkylsilane (Sigma), a slide glass treated with polylysine (Sigma) and a slide glass treated with aminoalkylsilane and glutaraldehyde. The slide glass treated with aminoalkylsilane and glutaraldehyde was prepared by soaking the slide glass treated with aminoalkylsilane in a 2.5% glutaraldehyde aqueous solution for 1 hour, washing it three times in distilled water and air-drying it.

On the other hand, immobilization was similarly carried out using a slide glass without treatment as a control.

The immobilization rates were determined as described in Example 1. The results are shown in Table 4.

Table 4

| Surface treatment of support | Solvent for DNA | | Immobilization rate (%) |
|---|---|---|---|
| No treatment | 50mM NMM buffer | (pH 8.0) | 30.7 |
| | | (pH 9.0) | 20.4 |
| | 50mM Carbonate buffer | (pH9.5) | 5.1 |
| | | (pH10.5) | 2.3 |
| Aminoalkylsilane | 50mM NMM buffer | (pH 8.0) | 41.0 |
| | | (pH 9.0) | 58.6 |
| | 50mM Carbonate buffer | (pH 9.5) | 30.2 |
| | | (pH10.5) | 20.8 |
| Aminoalkylsilane and glutaraldehyde | 50mM NMM buffer | (pH 8.0) | 48.5 |
| | | (pH 9.0) | 52.7 |
| | 50mM Carbonate buffer(pH | 9.5) | 16.7 |
| | | (pH10.5) | 16.7 |
| Polylysine | 50mM NMM buffer | (pH 8.0) | 45.2 |
| | | (p H9.0) | 77.1 |
| | 50mM Carbonate buffer(pH | 9.5) | 27.2 |
| | | (pH10.5) | 31.0 |

As shown in Table 4, it was confirmed that the immobilization rates observed for the slide glasses treated with aminoalkylsilane or polylysine were increased as compared with the immobilization rate observed for the slide glass without treatment. Also, it was confirmed that the immobilization rate observed for the slide glass treated with aminoalkylsilane and glutaraldehyde was increased as compared with the immobilization rate observed for the slide glass without treatment.

Example 4

Immobilization of oligonucleotide

An oligonucleotide of 20 bases in length which has the same base sequence as that of the primer of SEQ ID NO:1 used in Example 1 and to which ROX is attached at the 5'-terminus (hereinafter referred to as ROX oligo), and an oligonucleotide to which an alkylamino linker (PE Biosystems) which has an alkylene chain of 6 and ROX are attached at the 5'-terminus and at the 3'-termunus, respectively (hereinafter referred to as ROX amino oligo) were synthesized using a DNA synthesizer. These synthetic DNAs were dissolved in 50 mM N-methylmorpholine-hydrochloride (NMM) buffer (pH 9.5) or 50 mM carbonate buffer (pH 9.5) at a concentration of 10 μM and used for examination of immobilization onto slide glasses which had been subjected to various surface treatment as described in Example 3.
On the other hand, immobilization was examined for a DNA dissolved in TE as a control.
Slide glasses treated with aminoalkylsilane (AAS) and slide glasses treated with aminoalkylsilane and glutaraldehyde were used.
In this Example, the slide glass to which DNAs had been immobilized and which was then washed was further soaked in a 3:1 mixture of PBS - 100% ethanol containing 0.25% sodium boron hydride (Wako Pure Chemical Industries) for 5 minutes, and then washed and dried as described above in order to reduce the Schiff base formed as a result of the covalent bond between the aldehyde group on the support and the amino group in the DNA to a stable amine.
Immobilization rates were determined as described in Example 3. The results are shown in Table 5.

Table 5

| Solvent for DNA | Type of DNA | Surface treatment of support | Immobilization rate (%) |
|---|---|---|---|
| TE | ROX oligo | AAS | 0.7 |
| | | AAS + GA | 1.4 |
| | ROX amino oligo | AAS | 0.9 |
| | | AAS + GA | 1.6 |
| NMM buffer | ROX oligo | AAS | 5.8 |
| | | AAS + GA | 13.2 |
| | ROX amino oligo | AAS | 19.1 |
| | | AAS + GA | 65.6 |
| Carbonate buffer | ROX oligo | AAS | 4.4 |
| | | AAS + GA | 7.7 |
| | ROX amino oligo | AAS | 16.6 |
| | | AAS + GA | 25.7 |
| AAS: aminoalkylsilane; GA: glutaraldehyde. | | | |

As shown in Table 5, it was confirmed that the use of either NMM buffer or carbonate buffer increased the immobilization rate for the combination of the ROX amino oligo and the slide glass treated with aminoalkylsilane and glutaraldehyde. On the other hand, the immobilization rate for the control in which TE was used was low regardless of the type of the immobilized oligonucleotide or the surface treatment of the support.

Example 5

Detection of target nucleic acid by hybridization

Detection of a target nucleic acid by hybridization was examined.

(1) Immobilization of DNA

Using the plasmid DNA containing human transferrin gene (4738 bp) used in Example 1 as a template, DNA fragments of 0.1 kb, 0.2 kb, 0.5 kb, 1.0 kb and 1.5 kb were PCR-amplified using primer pairs having base sequences of SEQ ID NOS:1 and 3, 1 and 4, 1 and 5, 1 and 2 or 1 and 6, respectively. Each of the PCR amplification products was dissolved in 50 mM N-methylmorpholine-hydrochloride buffer (pH 9.5) at a concentration of 1 μM. As controls, DNA solutions were prepared in a similar manner using 3 x SSC or TE. 0.2 μl each of the resulting DNA solutions was spotted onto a slide glass treated with aminoalkylsilane. The slide glass was subjected to immobilization as described in Example 1 for immobilizing the DNAs.

(2) Hybridization with target nucleic acid

An oligonucleotide having the base sequence of SEQ ID NO:1 and labeled with ROX at the 5'-terminus was chemically synthesized. The oligonucleotide was dissolved in 4 x SSC containing 0.2% SDS at a concentration of 1 μM. The resulting solution was used as a target nucleic acid solution. The solution was heated at 95°C for 2 minutes and then rapidly cooled. 3 to 5 μl of the solution was spotted onto the slide glass onto which DNAs had been immobilized in (1) above. The slide glass was covered with a cover glass such that no air bubble was included. The sides were shut tightly with a seal and the slide glass was incubated at 37 to 40°C for 2 to 6 hours. After removing the seal, the slide glass was washed in 0.2 x SSC containing 0.1% SDS for 30 minutes followed by 0.2 x SSC for 30 minutes and dried.

(3) Detection of target nucleic acid

The slide glass was set in FMBIO II Multi-View for reading the image and measuring the fluorescence intensity. The results are shown in Table 6.

Table 6

| Immobilized DNA | | Fluorescence intensity for target nucleic acid (peak area) |
|---|---|---|
| Solvent | Length (kb) | |
| 3 x SSC | 0.1 | 20 |
| | 0.2 | 57 |
| | 0.5 | 35 |
| | 1.0 | 43 |
| | 1.5 | 57 |
| TE | 0.1 | 23 |
| | 0.2 | 15 |
| | 0.5 | 14 |
| | 1.0 | 23 |
| | 1.5 | 19 |
| NMM buffer | 0.1 | 34 |
| | 0.2 | 99 |
| | 0.5 | 97 |
| | 1.0 | 79 |
| | 1.5 | 81 |

As shown in Table 6, it was confirmed that the fluorescence intensity after hybridization for the DNA spotted in NMM buffer was remarkably increased as compared with those observed using SSC or TE.

Example 6

Effect of addition of surfactant on spotting

A microarray was prepared by spotting DNA solutions containing various surfactants (cationic, anionic and non-ionic) to study the effects of the added surfactant on the amount of a spotted DNA solution and the shape of a dot.

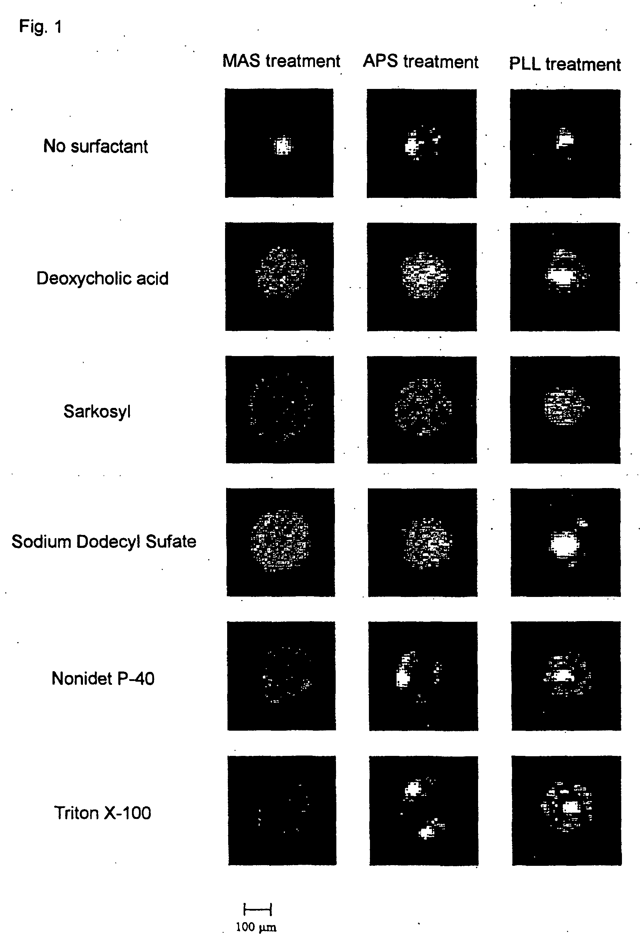

Briefly, DNA solutions containing a fluorescent substance were prepared by mixing, at final concentrations, 0.15 mg/ml salmon sperm DNA (Funakoshi), 0.1 μM Cy5-dUTP (Amersham Pharmacia), 200 mM sodium hydroxide, 300 mM sodium acetate and 0.1% of one of the various surfactants as shown in Table 7. Furthermore, a DNA solution containing a fluorescent substance which had the same composition as that described above except that no surfactant was added was prepared as a control. For each of the DNA solutions, 40 dots were spotted onto a surface of MAS-coated slide glass (Matsunami Glass, hereinafter referred to as MAS), aminopropyltriethoxysilane (APS)-coated slide glass (Matsunami Glass, hereinafter referred to as APS or poly-L-lysine (PLL)-coated slide glass (Matsunami Glass, hereinafter referred to as PLL) using GMS 417 Arrayer (Genetic Microsystems) equipped with a pin having a tip diameter of 130 μm. The slide glasses immediately after spotting were read using a confocal laser fluorescence microscope GMS 418 Array Scanner (Genetic Microsystems) for evaluating the shapes of dots. The fluorescence intensities for the dots were determined using an image analysis software ImaGene (BioDiscovery) based on the image read as described above. Average of the signal intensities determined for 40 spots was calculated. The calculated values are shown in Table 7. The typical shapes of the respective spots are shown in Figure 1. No fluorescence was detected for the dots when DNA solutions prepared as described above except that no Cy5-dUTP was added were spotted and the signals were read with the same detection sensitivity using GMS 418 Array Scanner.

Table 7

| Surfactant | Fluorescence intensity | | |
|---|---|---|---|
| | MAS | APS | PLL |
| No surfactant | 10980 | 9152 | 6562 |
| Cetyltrimethylammonium bromide (CTAB) | 3228 | 2611 | 1847 |
| Deoxycholic acid | 18745 | 17756 | 17030 |
| Benzalkonium chloride | 1644 | 2653 | 2206 |
| Sarkosyl | 15316 | 17592 | 17262 |
| Sodium dodecyl sulfate (SDS) | 22602 | 21305 | 15395 |
| Nonidet P-40 | 15914 | 12383 | 15401 |
| Triton X-100 | 13151 | 10427 | 14327 |
| Tween-20 | 11187 | 6579 | 10260 |

As seen from Table 7, for each of the surface-treated slide glasses, the fluorescence intensity for a dot (i.e., the amount of a spotted DNA solution) was increased only when a certain surfactant was added as compared with the intensity observed when no surfactant was added as a control. For example, the amount was increased when deoxycholic acid, Sarkosyl, SDS, Nonidet P-40 or Triton X-100 was added, whereas the amount was not increased when CTAB or benzalkonium chloride was used. The amount for the APS slide glass was not increased when Tween-20 was used. As shown in Figure 1, increase in spot size was observed for a dot for which increase in the amount of a spotted DNA solution was observed, indicating increase in number of effective pixels for signal detection. Heterogeneity of signal intensity in a spot was observed for some of the surfactants or slide glasses.

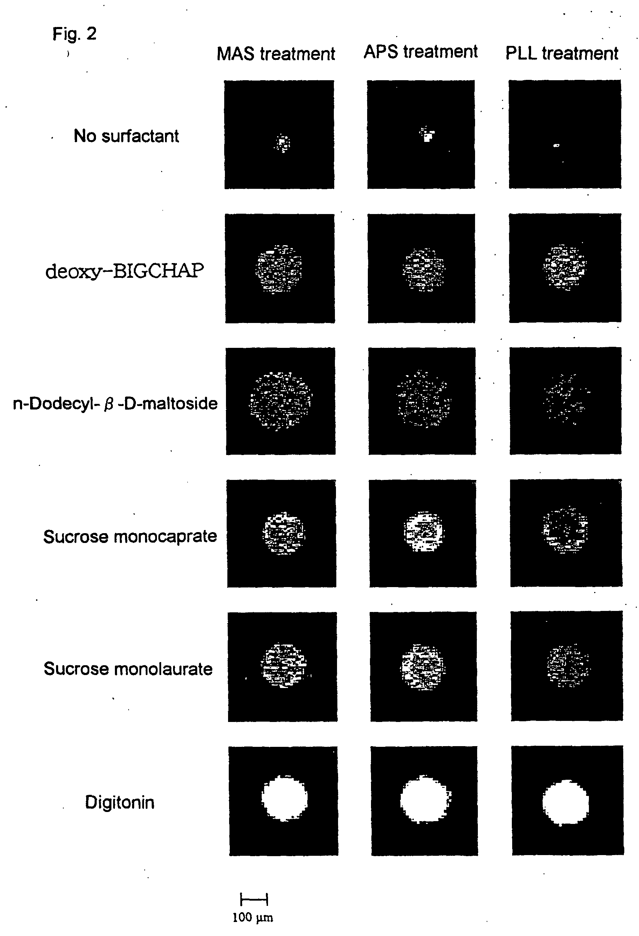

More types of surfactants were further examined. Specifically, the amounts of spotted DNA solutions and the shapes of dots were studied as described above using mainly non-ionic surfactants as shown in Table 8. The results are summarized in Table 8 and Figure 2. Using these surfactants, no obvious spot was also detected when no Cy5-dUTP was added.

Table 8

| Surfactant | Fluorescence intensity | | |
|---|---|---|---|
| | MAS | APS | PLL |
| No surfactant | 18118 | 7932 | 3417 |
| CHAPS | 24012 | 12669 | 14736 |
| CHAPSO | 26540 | 14586 | 17014 |
| BIGCHAP | 21554 | 11082 | 12491 |
| Deoxy-BIGCHAP | 31194 | 19128 | 19539 |
| n-Octyl-β-D-glucoside | 16865 | 8909 | 7086 |
| n-Heptyl-β-D-thioglucoside | 28069 | 14120 | 14058 |
| n-Octyl-β-D-thioglucoside | 45245 | 19961 | 21298 |

Table 8   (continued)

| Surfactant | Fluorescence intensity | | |
|---|---|---|---|
| | MAS | APS | PLL |
| n-Dodecyl-β-D-maltoside | 31913 | 20254 | 16669 |
| MEGA-8 | 12441 | 3424 | 5416 |
| MEGA-9 | 14347 | 4491 | 6524 |
| MEGA-10 | 22524 | 14022 | 11681 |
| Sucrose monocaprate | 34637 | 25135 | 21985 |
| Sucrose monolaurate | 34299 | 23625 | 17233 |
| Sodium cholate | 31748 | 17260 | 18550 |
| Digitonin | 56038 | 57063 | 51221 |

As seen from Table 8, the amount of a spotted DNA solution was increased in almost all cases where DNA solutions containing a fluorescent substance to which surfactants were added were spotted as compared with the control in which no surfactant was added regardless of the type of the slide glass. As shown in Figure 2, increase in spot size was observed for a spot for which increase in the amount of a spotted DNA solution was observed, indicating increase in number of effective pixels for signal quantification. Heterogeneity of signal intensity in a spot was decreased for all of the slide glasses when one of these surfactants was added. These results demonstrate that the amount of a DNA solution retained on a support is increased and that homogeneous dots, which are effective for quantifying signals derived from the retained DNAs, are formed by adding such a surfactant to a DNA solution.

Example 7

Effect of addition of surfactant on immobilization of PCR amplification product and sensitivity of detecting target nucleic acid

The effects of the several surfactants, which were shown to be remarkably effective in improving the shape of a dot and the amount of a spotted DNA solution in Example 6, on efficiency of immobilizing a nucleic acid and sensitivity of detecting a target nucleic acid by hybridization with an immobilized nucleic acid were examined.

(1) Preparation of fluorescence-labeled DNA fragment

A 0.2-kb DNA fragment was amplified by PCR using the plasmid DNA containing human transferrin gene (4738 bp) used in Example 1 as a template and primers having base sequences of SEQ ID NOS:1 and 4. One of the two primers used for the PCR, the primer of SEQ ID NO:1, was used after fluorescence-labeling with Cy3 at the 5'-terminus. The PCR amplification product was purified by isopropanol precipitation and then lyophilized.

(2) Preparation of DNA solution

DNA solutions each containing one of the various surfactants as shown in Table 9 were prepared so as to have the following compositions. Specifically, the fluorescence-labeled PCR fragment prepared as described above was dissolved at a concentration of 0.15 µg/µl in a solution containing 0.1% of one of the surfactants, 200 mM sodium hydroxide and 30 mM sodium acetate (pH 5.2). The resulting solutions were used as DNA solutions. A DNA solution without a surfactant was prepared as a control.

(3) Spotting and immobilization of DNA

The DNA solutions were spotted onto an MAS-, APS-or PLL-coated slide glass as described in Example 6. After spotting, the slide glasses were incubated at 37°C for 1 hour in a humidified incubator, irradiated with ultraviolet at 60 mJ/cm$^2$, washed in 0.2% SDS solution once followed by distilled water twice, and then centrifuged at low speed to remove water. The slide glasses were then soaked in a blocking solution for 20 minutes to block free amino groups. The blocking solution was prepared immediately before use by dissolving 2.75 g of succinic anhydride in 160 ml of N-methyl-2-pyrrolidone and further adding 15 ml of 1 M borate buffer (pH 8.0) thereto. Finally, the slide glasses were washed in distilled water twice, centrifuged to remove water and stored in a desiccator.

(4) Preparation of target nucleic acid

A 1-kb fragments for human transferrin receptor gene was amplified as described in Example 1 and purified by isopropanol precipitation. The DNA fragment was fluorescence-labeled with Cy5 using Label IT Cy5 Labeling Kit (Mirus) according to the instructions attached to the kit and then purified.

(5) Hybridization with target nucleic acid

The above-mentioned Cy5-labeled DNA fragment was denatured and neutralized using a denaturation solution and a neutralization solution attached to Label IT Cy5 Labeling Kit according to the instructions attached to the kit. About 10 µl of the resulting solution was spotted onto regions in which the DNAs had been immobilized on each of the slide glasses prepared in (3) above. The spots were covered with a cover glass such that no air bubble was included. Each of the cover glass was sealed with paper bond (Kokuyo) and the slide glasses were incubated at 65°C for 12 hours in a humidified incubator. After removing the paper bond and the cover glasses, the slide glasses were washed in 2 x SSC at 60°C for 5 minutes followed by 0.2 x SSC containing 0.1% SDS at 60°C for 5 minutes. The slide glasses were then washed in 0.2 x SSC at room temperature and dried.

(6) Quantification of fluorescence intensity and calculation of immobilization rate

Detection of fluorescence image of a spot and quantification of fluorescence intensity were carried out as described in Example 6. The fluorescence detected at the wavelength for Cy3 and the fluorescence detected at the wavelength for Cy5 were analyzed as those derived from the immobilized DNA and the target nucleic acid being hybridized with the immobilized DNA, respectively. Immobilization rates were determined as described in Example 1. The results are shown in Tables 9 and 10. Table 9 shows the fluorescence detected at the wavelength for Cy5 which represents the amount of the target nucleic acid being hybridized with the DNA immobilized onto the slide glass. Table 10 shows the immobilization rate of the DNA onto the slide glass calculated based on the fluorescence intensity detected at the wavelength for Cy3.

Table 9

| Surfactant | Fluorescence intensity for target nucleic acid | | |
|---|---|---|---|
| | MAS | APS | PLL |
| No surfactant | 9115 | 1748 | 1939 |
| Deoxy-BIGCHAP | 6250 | 2769 | 7028 |
| n-Dodecyl-β-D-maltoside | 7242 | 12508 | 16962 |
| Sucrose monolaurate | 13577 | 17898 | 16433 |
| Digitonin | 9008 | 11431 | 13548 |
| SDS | 16027 | 21355 | 10746 |
| Sarkosyl | 11298 | 17612 | 15993 |
| Deoxycholic acid | 6391 | 3647 | 6919 |
| Tween-20 | 9343 | 3337 | 5370 |
| Sucrose monocaprate | 10666 | 5935 | 8655 |

Table 10

| Surfactant | Immobilization rate (%) | | |
|---|---|---|---|
| | MAS | APS | PLL |
| No surfactant | 53.1 | 42.9 | 46.9 |
| Deoxy-BIGCHAP | 49.0 | 11.7 | 87.7 |
| n-Dodecyl-β-D-maltoside | 43.8 | 45.8 | 64.6 |
| Sucrose monolaurate | 53.0 | 53.7 | 65.7 |
| Digitonin | 47.1 | 42.7 | 64.9 |
| SDS | 55.9 | 59.2 | 70.3 |
| Sarkosyl | 54.1 | 53.9 | 64.7 |

Table 10   (continued)

| Surfactant | Immobilization rate (%) | | |
|---|---|---|---|
| | MAS | APS | PLL |
| Deoxycholic acid | 48.8 | 23.3 | 90.1 |
| Tween-20 | 48.3 | 55.7 | 75.4 |
| Sucrose monocaprate | 51.1 | 46.7 | 65.6 |

As seen from Table 9, using the APS and PLL slide glasses, the effect of remarkably increasing the fluorescence intensity due to hybridrzation with the target nucleic acid was observed for all of the surfactants examined. Using the slide glass treated with MAS, a similar effect was observed when DNA solutions to which sucrose monolaurate, sucrose monocaprate, SDS or Sarkosyl

was added were spotted.

In addition, as seen from Table 10, no change in immobilization rate due to the presence of a surfactant was observed for the surfactants other than deoxy-BIGCHAP and deoxycholic acid as compared with the control with no addition.

Based on the findings obtained in Examples 6 and 7, it was demonstrated that non-ionic surfactants such as, in particular, sucrose monolaurate, sucrose monocaprate and digitonin can be preferably used to dramatically improve the shape of a dot, the amount of a spotted DNA solution and the sensitivity of detecting a target nucleic acid regardless of the type of surface treatment of a slide glass.

Example 8

Effect of concentration of surfactant on shape of dot and spotted amount

The effects of the concentrations of two non-ionic surfactants in DNA solutions on their effects were examined. The two non-ionic surfactants, i.e., sucrose monolaurate and digitonin, were judged to be preferably used to improve the amount of a spotted DNA solution and the shape of a dot in Examples 6 and 7. Briefly, DNA solutions for spotting each containing one of the surfactants at a varying concentration were prepared and spotted onto a MAS-, APS- or PLL-coated slide glasses as described in Example 7. The fluorescence intensity and the shape were evaluated for each dot immediately after spotting. The concentrations of the respective surfactants examined are shown in Table 11. Average of the fluorescence intensities determined for 40 spots was calculated. The calculated values are shown in Table 11.

Table 11

| DNA solution | | Fluorescence intensity | | |
|---|---|---|---|---|
| Surfactant | Concentration (%) | MAS | APS | PLL |
| No surfactant | 0.000 | 8750 | 7437 | 5016 |
| Sucrose monolaurate | 0.100 | 15021 | 19053 | 16513 |
| | 0.050 | 15668 | 19474 | 17516 |
| | 0.025 | 13443 | 14742 | 12777 |
| | 0.013 | 11688 | 13380 | 10249 |
| | 0.001 | 10846 | 10817 | 6580 |
| Digitonin | 0.100 | 10729 | 12243 | 12026 |
| | 0.050 | 10428 | 11399 | 9858 |
| | 0.025 | 10401 | 11554 | 9485 |
| | 0.013 | 8683 | 10164 | 8227 |
| | 0.001 | 8624 | 8737 | 5504 |

As shown in Table 11, an effect of increasing the amount of a spotted DNA solution was observed for each slide glass when sucrose monolaurate was added at a concentration ranging from 0.001% to 0.1%. The effect was observed when digitonin was added at a concentration ranging from 0.025% to 0.1% (MAS), from 0.001% to 0.1% (APS), or from 0.013% to 0.1% (PLL). The shape of the dot had a tendency to be improved as the amount of a spotted DNA solution was increased.

The slide glasses were subjected to immobilization, hybridization with a target nucleic acid and detection of signals as described in Example 7. As a result, the amount of the immobilized DNA in each dot after hybridization was constant without being influenced by the concentration of the surfactant. The shape of hybridization signal had a tendency to be similar to that observed immediately after spotting.

Industrial Applicability

As described above, according to the method of the present invention, a larger amount of a DNA can be immobilized onto a solid support as compared with the amount accomplished by a conventional method. Immobilization rate to a support or density of an immobilized DNA can be increased according to the method of the present invention. Furthermore, detection sensitivity in a method for detecting a target nucleic acid can be increased by using a material onto which a DNA is immobilized prepared according to the immobilization method of the present invention.

Sequence Listing Free Text

SEQ ID NO: 1: Designed oligonucleotide to amplify a portion of transferrin receptor gene.
SEQ ID NO: 2: Designed oligonucleotide to amplify a portion of transferrin receptor gene.
SEQ ID NO: 3: Designed oligonucleotide to amplify a portion of transferrin receptor gene.
SEQ ID NO: 4: Designed oligonucleotide to amplify a portion of transferrin receptor gene.
SEQ ID NO: 5: Designed oligonucleotide to amplify a portion of transferrin receptor gene.
SEQ ID NO: 6: Designed oligonucleotide to amplify a portion of transferrin receptor gene.

**Claims**

1. A method for immobilizing a DNA onto a support, the method comprising contacting a DNA with a support in a buffer containing at least one substance selected from the group consisting of morpholine, a morpholine derivative, a salt thereof and a carbonate.

2. The method according to claim 1, wherein the morpholine derivative is an N-alkylmorpholine substituted with a C1-3 alkyl group.

3. The method according to claim 1, wherein the salt of morpholine or the morpholine derivative is a salt selected from the group consisting of a salt with a mineral acid, a salt with an organic acid and a salt with a fatty acid.

4. The method according to claim 1, wherein the carbonate is a salt selected from the group consisting of a sodium salt, a potassium salt, a magnesium salt, an ammonium salt and a triethylamine salt.

5. The method according to claim 1, wherein the buffer contains at least one substance selected from the group consisting of morpholine, a morpholine derivative and a salt thereof in combination with at least one carbonate.

6. The method according to any one of claims 1 to 5, wherein the pH of the buffer is 7 to 11.

7. The method according to any one of claims 1 to 6, wherein the concentration of at least one substance selected from the group consisting of morpholine, a morpholine derivative, a salt thereof and a carbonate in the buffer is 10 to 500 mM.

8. The method according to any one of claims 1 to 7, wherein the DNA is an oligonucleotide, a polynucleotide or a derivative thereof.

9. The method according to any one of claims 1 to 8, wherein the concentration of the DNA in the buffer is 0.1 to 2.0 mg/ml.

**10.** The method according to any one of claims 1 to 9, wherein the buffer further contains at least one salt.

**11.** The method according to any one of claims 1 to 10, wherein the buffer further contains at least one surfactant.

**12.** The method according to any one of claims 1 to 11, wherein the support is made from glass or quartz, or is a material prepared by treating the surface of glass or quartz.

**13.** The method according to claim 12, wherein the surface is treated with a silane coupling agent or a polycation.

**14.** The method according to any one of claims 1 to 13, wherein the DNA is immobilized without denaturation.

**15.** The method according to claim 14, wherein the DNA is a double-stranded DNA.

**16.** A method for immobilizing a DNA onto a support, the method comprising contacting a DNA with a support in a buffer containing at least one surfactant.

**17.** The method according to claim 16, wherein the surfactant is selected from the group consisting of a non-ionic surfactant, an anionic surfactant and an amphoteric surfactant.

**18.** The method according to claim 17, wherein the surfactant is selected from the group consisting of sucrose monocaprate, sucrose monolaurate and digitonin.

**19.** The method according to any one of claims 16 to 18, wherein the buffer contains at least one substance selected from the group consisting of morpholine, a morpholine derivative, a salt thereof and a carbonate.

**20.** The method according to claim 19, wherein the pH of the buffer is 7 to 11.

**21.** The method according to claim 19 or 20, wherein the concentration of at least one substance selected from the group consisting of morpholine, a morpholine derivative, a salt thereof and a carbonate in the buffer is 10 to 500 mM.

**22.** The method according to any one of claims 16 to 21, wherein the DNA is an oligonucleotide, a polynucleotide or a derivative thereof.

**23.** The method according any one of claims 16 to 22, wherein the concentration of the DNA in the buffer is 0.1 to 2.0 mg/ml.

**24.** The method according to any one of claims 16 to 23, wherein the buffer further contains at least one salt.

**25.** The method according to any one of claims 16 to 24, wherein the support is made from glass or quartz, or is a material prepared by treating the surface of glass or quartz.

**26.** The method according to claim 25, wherein the surface is treated with a silane coupling agent or a polycation.

**27.** The method according to any one of claims 16 to 26, wherein the DNA is immobilized without denaturation.

**28.** The method according to claim 27, wherein the DNA is a double-stranded DNA.

**29.** A material onto which a DNA is immobilized, which is prepared according to the method defined by any one of claims 1 to 28.

**30.** The material according to claim 29, wherein the DNA is a double-stranded DNA.

**31.** A method for detecting a target nucleic acid, the method comprising detecting a target nucleic acid by using the material onto which a DNA is immobilized defined by claim 29 or 30.

**32.** The method according to claim 31, comprising hybridizing the material onto which a DNA is immobilized with the target nucleic acid under stringent conditions.

**33.** The method according to claim 32, wherein the DNA immobilized on the material is hybridized with the target nucleic acid under stringent conditions without denaturation of the immobilized DNA.

**34.** A material in which a double-stranded DNA is immobilized onto a support, which can be used for hybridization with a target nucleic acid under stringent conditions without denaturing the immobilized DNA.

**35.** A buffer for immobilizing a DNA, which contains at least one substance selected from the group consisting of morpholine, a morpholine derivative, a salt thereof and a carbonate.

**36.** A buffer for immobilizing a DNA, which contains at least one surfactant.

**37.** A buffer for immobilizing a DNA, which contains at least one substance selected from the group consisting of morpholine, a morpholine derivative, a salt thereof and a carbonate as well as at least one surfactant.

**Fig. 1**

MAS treatment   APS treatment   PLL treatment

No surfactant

Deoxycholic acid

Sarkosyl

Sodium Dodecyl Sufate

Nonidet P-40

Triton X-100

100 μm

**Fig. 2**

|  | MAS treatment | APS treatment | PLL treatment |
| --- | --- | --- | --- |

No surfactant

deoxy–BIGCHAP

n-Dodecyl- $\beta$ -D-maltoside

Sucrose monocaprate

Sucrose monolaurate

Digitonin

100 μm

## INTERNATIONAL SEARCH REPORT

| International application No. |
| --- |
| PCT/JP99/06865 |

| A. CLASSIFICATION OF SUBJECT MATTER |
| --- |
| Int.Cl⁷ C12N 15/00, C12M 1/00, C12Q 1/68, G01N 33/53, G01N 33/50 |

According to International Patent Classification (IPC) or to both national classification and IPC

| B. FIELDS SEARCHED |
| --- |
| Minimum documentation searched (classification system followed by classification symbols)<br>Int.Cl⁷ C12N 15/00, C12M 1/00, C12Q 1/68, G01N 33/53, G01N 33/50 |
| Documentation searched other than minimum documentation to the extent that such documents are included in the fields searched |
| Electronic data base consulted during the international search (name of data base and, where practicable, search terms used)<br>WPI(DIALOG), BIOSIS(DIALOG) |

C. DOCUMENTS CONSIDERED TO BE RELEVANT

| Category* | Citation of document, with indication, where appropriate, of the relevant passages | Relevant to claim No. |
| --- | --- | --- |
| Y | JP, 9-322777, A (Japan Synthetic Rubber Co., Ltd.), 16 December, 1997 (16.12.97) (Family: none) | 1-37 |
| Y | JP, 7-132077, A (HAMAMATSU PHOTONICS K.K.), 23 May, 1995 (23.05.95) (Family: none) | 1-37 |
| Y | EP,814156,A2(THE INSTITUTE OF PHYSICAL & CHEMICAL RESEARCH) especially, claim 13 and page 3, lines 32~38 & JP,10-155481,A especially, claim 13 and Par. No. [0013] & US,5916775,A | 1-37 |
| Y | EP, 821069, A1 (UNITIKA LTD.), 28 January, 1998 (28.01.98), especially, page 5, lines 15 to 38 & JP, 10-33196, A especially, Par. Nos. [0035] to [0038] & US, 5912139, A | 1-37 |
| Y | EP, 346865, A (DU PONT DE NEMOURS & CO.E I), | 1-37 |

☒ Further documents are listed in the continuation of Box C.  ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>13 March, 2000 (13.03.00) | Date of mailing of the international search report<br>21 March, 2000 (21.03.00) |
| --- | --- |
| Name and mailing address of the ISA/<br>Japanese Patent Office | Authorized officer |
| Facsimile No. | Telephone No. |

Form PCT/ISA/210 (second sheet) (July 1992)

22

## INTERNATIONAL SEARCH REPORT

International application No.

PCT/JP99/06865

| | C (Continuation). DOCUMENTS CONSIDERED TO BE RELEVANT | |
|---|---|---|
| Category* | Citation of document, with indication, where appropriate, of the relevant passages | Relevant to claim No. |
| | 20 December, 1989 (20.12.89),<br>especially, page 5, lines 6 to 7<br>& JP, 2-109979, A<br>especially, page 6, upper right column, line 17 to page<br>6, lower left column, line 3 | |

Form PCT/ISA/210 (continuation of second sheet) (July 1992)